# EUROPEAN PATENT APPLICATION

(11) **EP 1 666 036 A1**
(43) Date of publication of application: **07.06.2006**
(21) Application number: 04773485.0
(22) Date of filing: 22.09.2004
(51) Int. Cl.: A61K 31/353, A61K 8/18, A23L 1/30, A61P 17/14, C07D 311/18

(54) **HAIR RESTORER**

(30) Priority: 22.09.2003 JP 2003329553
(71) Applicant: Nippon Shinyaku Co., Ltd., Kyoto-shi, Kyoto 601-8550 (JP); SUNSTAR INC., Takatsuki-shi, Osaka 569-1195 (JP)
(72) Inventor: NAKAOJI, Kouichi, c/o SUNSTAR INC., Takatsuki-shi, Osaka 569-1195 (JP); MATSUURA, Masahiro, c/o SUNSTAR INC., Takatsuki-shi, Osaka 569-1195 (JP); NISHI, Toyoyuki, 6210862 (JP)
(74) Representative: Müller-Boré & Partner Patentanwälte
(86) International application number: PCT/JP2004/014311
(87) International publication number: WO 2005/027904

(57) **Abstract**

The present invention provides a hair growth stimulant comprising a chromene compound as an active ingredient, and which exhibits excellent hair restoration effects. The present invention also provides a hair-restoring preparation for external use, hair-restoring preparation for oral use, and hair-restoring food containing the hair growth stimulant.

## Description

### TECHNICAL FIELD

The present invention relates to a hair growth stimulant containing a chromene compound as an active ingredient. The present invention also relates to a hair-restoring preparation for external use, a hair-restoring preparation for oral use, and a hair-restoring food containing the hair growth stimulant.

### BACKGROUND OF THE INVENTION

Recently, an increasing number of men and women are suffering from thinning hair or hair loss caused by various changes in their social environment, such as an increase in stress factors and changes in eating habits, and thus the social expectations and needs for hair growth stimulants have been increasing. Hitherto, hair growth stimulants containing a variety of active ingredients that are intended to eliminate or alleviate the causes of thinning hair or hair loss have been developed. For example, hair growth stimulants containing a swertia herb extract or tocopherol acetate to increase the blood flow to the hair roots, and hinokitiol to improve scalp metabolism have been developed and used to prevent or treat alopecia. It is believed, however, that various factors, such as genetic predisposition, stress, eating habits and aging, are combined in a complicated manner to cause thinning hair or hair loss. Thus, no satisfactory effects of hair loss prevention or hair regrowth can be obtained simply by adding ingredients to improve blood flow or scalp metabolism, like hitherto known hair growth stimulants (see Fragrance Journal, Vol. 21, No. 9, pp. 37-42, 1993).

A chromene compound is known as a compound having an antibacterial effect or an antihypertensive effect. For example, Japanese Unexamined Patent Publication No.1982-28080 discloses that daurichromenic acid derived from Dahurian Azalea *(Rhododendron dauricum)* and derivatives thereof are useful as an antibacterial agent or a smooth muscle relaxant. Japanese Unexamined Patent Publication Nos. 1983-201776, 1984-093076, 1984-219277, 1984-219278, and 1986-012685 disclose that a chromene compound has antihypertensive effects. Japanese Unexamined Patent Publication No. 1988-130590 discloses that a chromene compound has the effect of inhibiting lipid peroxide formation. Japanese Unexamined Patent Publication No. 1989-199957 discloses that a chromene compound has a therapeutic effect for wounds and an anti-allergy effect. A national publication of the translated version of PCT application No.1993-506844 discloses that chromene derivatives are useful for preventing or treating hypercholesterolemia, atherosclerosis and other diseases caused by hypercholesterolemia and/or atherosclerosis. Furthermore, acute toxicity of methylripariochromene A, which is a chromene compound, was examined and it was reported that methylripariochromene A has no toxicity (see Annual Report of Toyama Prefectural Institute for Pharmaceutical Research, No. 1997 (25), pp. 23-35, 1998). As described above, chromene compounds have been studied as safe materials that are useful as pharmaceutical preparations. However, the hair restoration effects of chromene compounds have not been clarified.

### DISCLOSURE OF THE INVENTION

One of main objects of the present invention is to provide a hair growth stimulant having excellent hair restoration effects, such as the prevention of hair loss, promotion of hair growth and to provide an effective application form of the hair growth stimulant.

The present inventors conducted extensive research to achieve the above objects and found that a chromene compound having a specific structure has excellent hair restoration effects. The present invention was accomplished based on this finding.

In other words, the present invention provides a hair growth stimulant comprising at least one chromene compound represented by Structural Formula (1) as an active ingredient, and provides a preparation for external use, a preparation for oral use, and a food containing the hair growth stimulant. '

Item 1. A hair growth stimulant comprising at least one chromene compound represented by Structural Formula (1) as an active ingredient: 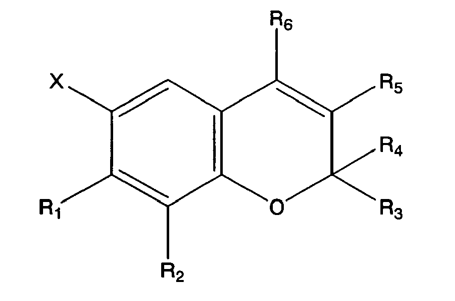 wherein R₁ and R₂ are each independently -H, -OH, or -OR wherein R is a C₁₋₄ alkyl group; R₃ and R₄ are each independently -H, or a C₁₋4 alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group.

Item 2. The hair growth stimulant according to Item 1, wherein the chromene compound represented by Structural Formula (1) is at least one member selected from the group consisting of methylripariochromene A (6-acetyl-7,8-dimethoxy-2,2-dimethylchromene), acetovanillochromene (6-acetyl-8-methoxy-2,2-dimethylchromene), and orthochromene A (6-(1-hydroxyethyl)-7,8-dimethoxy-2,2-dimethylchromene).

Item 3. The hair-restoring preparation for external use comprising the hair growth stimulant of Item 1 or 2.

Item 4. The hair-restoring preparation for oral use comprising the hair growth stimulant of Item 1 or 2.

Item 5. The hair-restoring food comprising the hair growth stimulant of Item 1 or 2.

Item 6. A method for preparing a hair growth stimulant containing at least one chromene compound represented by Structural Formula (1) as an active ingredient, 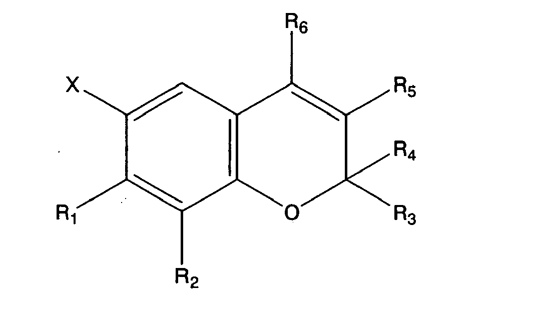 wherein R₁ and R₂ are each independently -H, -OH, or -OR wherein R is a C₁₋₄ alkyl group; R₃ and R₄ are each independently -H, or a C₁₋₄ alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group; the method comprising steps (i) and (ii):
(i) obtaining a chromene compound-containing extract by contacting a solvent with at least one plant selected from the group consisting of plants of the genus *Orthosiphon* in the family Lamiaceae, plants of the genus *Eupatorium* in the family Asteraceae, and plants of the genus Stevia in the family Asteraceae; and
(ii) isolating a chromene compound represented by Structural Formula (1) from the extract obtained in (i).

Item 7. The method for preparing a hair growth stimulant according to Item 6, wherein the chromene compound represented by Structural Formula (1) is at least one member selected from the group consisting of methylripariochromene A (6-acetyl-7,8-dimethoxy-2,2-dimethylchromene), acetovanillochromene (6-acetyl-8-methoxy-2,2-dimethylchromene), and orthochromene A (6-(1-hydroxyethyl)-7,8-dimethoxy-2,2-dimethylchromene).

Item 8. A hair-restoring method comprising the step of contacting an effective amount of a hair growth stimulant containing at least one chromene compound represented by Structural Formula (1) as an active ingredient with hair tissue: 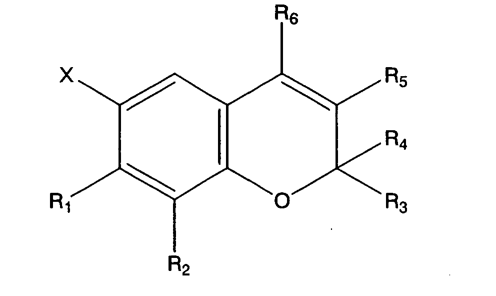 wherein R₁ and R₂ are each independently -H, -OH, or -OR wherein R is a C₁₋₄ alkyl group; R₃ and R₄ are each independently -H, or a C₁₋₄ alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group.

### Detailed Description of the Invention

The present invention is explained in more detail below.

In this specification, "%" indicates "weight %", unless otherwise specified.

### Chromene compound

In the hair growth stimulant of the present invention, a chromene compound represented by Structural Formula (1) functions as an active ingredient.

### Structural Formula (1):

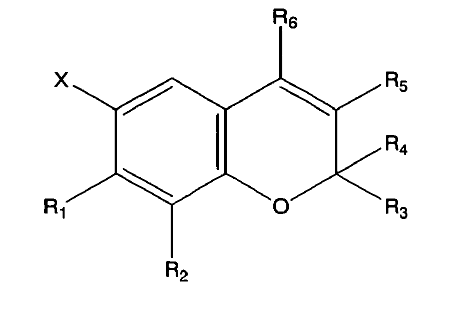

In Structural Formula (1), R₁ and R₂ are each independently -H, -OH, or -OR, and R is a C₁₋₄ alkyl group. The alkyl group includes straight-chain, and branched alkyl groups. Examples of -OR include -OCH₃, -OCH₂CH₃, -OCH₂CH₂CH₃, -OCH₂CH₂CH₂CH₃.

R₃ and R₄ are each independently -H, or a C₁₋₄ alkyl group. The alkyl group includes straight-chain, and branched alkyl groups. The C₁₋₄ alkyl group includes -CH₃, -CH₂CH₃, - CH(CH₃)₂.

R₅ and R₆ are each independently -H, a C₁₋₄ alkyl group, or an optionally substituted phenyl group. The alkyl group includes straight-chain, and branched alkyl groups. Examples of the C₁₋₄ alkyl group include -CH₃, -CH₂CH₃, -CH(CH₃)₂. Examples of an optionally substituted phenyl group include a phenyl group having one or more substituents, and a phenyl group without a substituent. The substituent may be, for example, a C₁₋₄ alkyl group. Specific examples of an optionally substituted phenyl group include -C₆H₅, -C₆H₄CH₃.

X is -CH(OH) R₇, or -C(=O)R₇.

R₇ is a C₁₋₄ alkyl group, or an optionally substituted phenyl group. The alkyl group includes straight-chain, and branched alkyl groups. Examples of the C₁₋₄ alkyl group include - CH₃, -CH₂CH₃, -CH(CH₃)₂. Examples of an optionally substituted phenyl group include a phenyl group having one or more substituents, and a phenyl group without a substituent. The substituent may be, for example, a C₁₋₄ alkyl group. Specific examples of an optionally substituted phenyl group include -C₆H₅, -C₆H₄CH₃.

Examples of compounds wherein X is -C(=O)R₇ include methylripariochromene A (6-acetyl-7,8-dimethoxy-2,2-dimethylchromene wherein R₁=-OCH₃, R₂=-OCH₃, R₃=-CH₃, R₄=-CH₃, R₅=-H, R₆=-H, and R₇=-CH₃; acetovanillochromene (6-acetyl-8-methoxy-2,2-dimethylchromene) wherein R₁=-H, R₂=-OCH₃, R₃=-CH₃, R₄=-CH₃, R₅=-H, R₆=-H, and R₇=-CH₃.

Examples of compounds wherein X is -CH(OH)R₇ include orthochromene A (6-(1-hydroxyethyl)-7,8-dimethoxy-2,2-dimethylchromene) wherein R₁=-OCH₃, R₂=-OCH₃, R₃=-CH₃, R₄=-CH₃, R₅=-H, R₆=-H, and R₇=-CH₃.

Methylripariochromene A is particularly preferable.

### Production method for a chromene compound

The chromene compound used in the present invention can be synthesized with a known method. The chromene compound used in the present invention can be obtained by isolating a chromene compound from a plant containing the chromene compound by, for example, extraction.

### Synthetic method

The chromene compound used in the present invention can be obtained by employing a known method to synthesize it. For example, methylripariochromene A can be obtained by the following synthetic pathway.

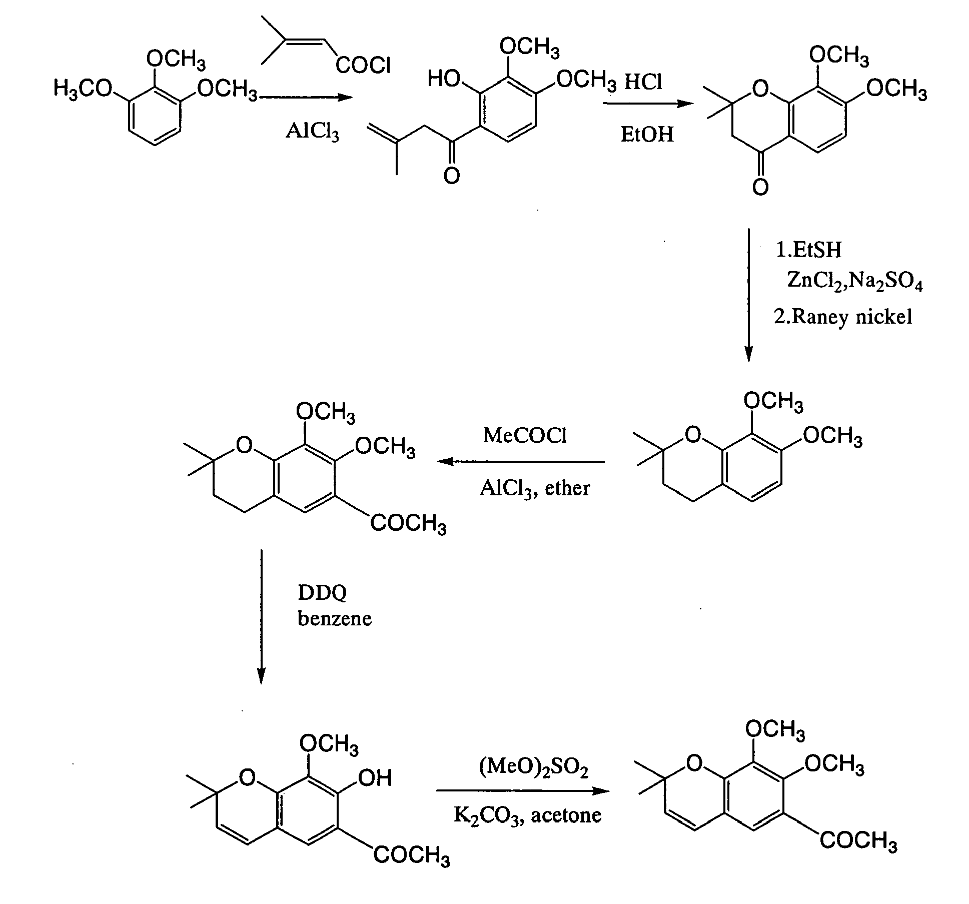

Chromene compounds represented by Structural Formula (1) having various substituents can be obtained by employing a synthetic method in the same manner as described above while using an intermediate having a desired substituent or suitably inserting a desired functional group.

The thus-obtained compounds can be suitably purified by employing a method of dissolution, extraction, liquid separation, slant, filtration, concentration, distillation, sublimation, or crystallization singly or in combination using an instrument such as a thin-layer chromatograph, column chromatograph, gas chromatograph and high precision liquid chromatograph.

### Method for isolating chromene compound from plants

It is also possible to obtain the chromene compound of the present invention by isolating the chromene compound from a plant containing the chromene compound by extraction, etc.

Examples of plants containing such a chromene compound include those of the genus *Orthosiphon* in the family Lamiaceae, the genuses *Eupatorium* and *Stevia* in the family Asteraceae.

Examples of plants of the genus *Orthosiphon* in the family Lamiaceae include Cat's whiskers *(Orthosiphon aristatus (Blume) Miq.), Orthosiph on grandiflorus Bold., Orthosiphon rubicundus Benth., Orthosiphon spicatus Benth., Orthosiphon stamineus Benth..*

Examples of plants of the genus *Eupatorium* in the family Asteraceae include *Eupatorium riparium Regel.*

Examples of the plants of the genus Stevia in the family Asteraceae include Stevia serrata Cav.

There are no limitations in the parts used to be extracted. For example, stalks, leaves, flowers and other above-ground parts; roots and other under-ground parts; whole plants, can be used. Above-ground parts are particularly preferable.

The material to be extracted may be a raw plant or a plant in a form of a dried product. The material plant may be pulverized before extraction.

The raw or dried extraction material is cut into pieces if necessary, extraction is conducted using a suitable solvent, and separation and purification are then conducted so that the chromene compound used in the present invention can be isolated.

There are no limitations on the extraction methods, and known methods such as batch methods, percolation methods and refluxing methods, can be used.

Extraction solvents may be suitably selected. Examples of usable solvents include water, various organic solvents, mixtures thereof. Examples of usable organic solvents include methanol, ethanol and like lower alcohols, chloroform, ethyl acetate, n-hexane.

The ratio of extraction solvent to plant material is not limited; however, it is preferable that about 2 to 1000 parts by weight of extraction solvent be used per part by weight of raw or dried plant (cut into pieces if necessary).

The extraction temperature may be room temperature or above, and the extraction temperature may be set, for example, in the range from room temperature to about 80°C.

The extraction operation may also be suitably selected. For example, extraction may be conducted in a temperature range from room temperature to about 80°C, for about 1 to 10 hours, while gently stirring. It is also possible to conduct extraction by placing the material to be extracted in a cylinder and adding a solvent dropwise to the material to be extracted.

### Hair growth stimulant

The hair growth stimulant of the present invention comprises at least one of the above-mentioned chromene compounds represented by Structural Formula (1) as an active ingredient. The hair growth stimulant of the present invention may contain one or more kinds of such chromene compounds.

The hair growth stimulant of the present invention is obtained by using just the chromene compound synthesized or isolated from a plant as described above, or by mixing the chromene compound with a material that can be used as a carrier, and being formed into various forms such as powders, blocks and liquids.

To be more specific, when a chromene compound isolated from a plant is used, the hair growth stimulant of the present invention can be produced by a method comprising the following steps (i) and (ii).

Step (i): obtaining a chromene compound-containing extract by contacting a solvent with at least one plant selected from the group consisting of plants of the genus *Orthosiphon* in the family Lamiaceae, plants of the genus *Eupatorium* in the family Asteraceae, and plants of the genus *Stevia* in the family Asteraceae.

Step (ii): isolating a chromene compound represented by Structural Formula (1) from the extract obtained in (i).

When the chromene compound obtained in Step (ii) is to be formed into a preparation, Step (iii) may be suitably added after Steps (i) and (ii).

Step (iii): forming the chromene compound isolated in Step (ii) into a preparation by using the chromene compound as it is or by being mixed with a material usable as a carrier.

In the above method for producing the hair growth stimulant, examples of usable chromene compounds represented by Structural Formula (1) include methylripariochromene A (6-acetyl-7,8-dimethoxy-2,2-dimethylchromene), acetovanillochromene (6-acetyl-8-methoxy-2,2-dimethylchromene), orthochromene A (6-(1-hydroxyethyl)-7,8-dimethoxy-2,2-dimethylchromene).

The hair growth stimulant of the present invention may comprise a suitable additive if necessary.

The hair growth stimulant of the present invention may be suitably formed into tablets, pulvis, capsules, etc.

Hair restoration can be conducted by contacting an effective amount of the hair growth stimulant of the present invention with hair tissue. In other words, the hair-restoring method of the present invention comprises a step of contacting an effective amount of hair growth stimulant with hair tissue.

The method for contacting the hair growth stimulant of the present invention with hair tissue is not limited. For example, such a method can be conducted by applying the hair growth stimulant of the present invention to hair tissue in the form of a preparation for external use that contains the hair growth stimulant of the present invention.

The effective amount can be suitably selected depending on the method of contact; age, gender and other conditions of the subject; etc.

Types of hair tissue are not limited either and include scalp, skin and other living body tissues, and hair-matrix cells and other living body cells.

By conducting the hair-restoring method of the present invention, hair loss prevention, and hair restoration effects such as hair regrowth and/or hair-nourishment can be achieved.

### Hair-restoring preparation for external use

The hair-restoring preparation for external use of the present invention is an external application that contains the hair growth stimulant of the present invention. The hair-restoring preparation for external use of the present invention can be obtained by forming the hair growth stimulant of the present invention by itself, or by suitably mixing it with known ingredients for external use medicines, into a preparation.

There are no limitations on the form of the hair-restoring preparation for external use of the present invention, and it may be formed into any type of preparation, including liquids, emulsions, creams, as long as it can be applied to the scalp or skin. The hair-restoring preparation for external use of the present invention may be suitably prepared into tonics, lotions, ointments, etc.

In addition to the hair growth stimulant of the present invention, the hair-restoring preparation for external use of the present invention may contain other ingredients that enhance hair regrowth and/or hair-nourishment effects. As examples of such other ingredients, the hair-restoring preparation for external use of the present invention may comprise one or more members selected from the group consisting of minoxidil, diazoxide, various antiandrogen agents (e.g., oxendolone, 4-androstene-3,17-dione-17-cyclic ethylene ketal derivatives), nicotinic acid and derivatives thereof, vitamin-E acetate, vitamin-E nicotinate, pantothenic acid and derivatives thereof, biotin, glycyrrhizinic acid, glycyrrhetic acid, plant worm extractives, *Panax ginseng* extracts, swertia herb extracts, capsicum extracts, cepharanthine, placental extracts, ethinylestradiol, carpronium chloride, photosensitive elements, other vitamins and amino acids.

The hair-restoring preparation for external use of the present invention may further comprise one or more additives generally used for hair-restoring preparations for external use selected from the group consisting of hinokitiol, hexachlorophene, phenol, benzalkonium chlorides, cetylpyridinium chloride, undecylic acid, trichlorocarbanilide, bithionol and like antibacterial agents; menthol and like refrigerants; salicylic acid, zinc and derivatives thereof; lactic acid, alkyl esters thereof and like medicaments; olive oil, squalane, liquid paraffin, isopropyl myristate, higher fatty acids, higher alcohols and like oils; and surfactants, fragrances, antioxidants, ultraviolet absorbers, pigments, ethanol, water, humectants, thickeners, solubilizing agents, in such an amount that does not adversely affect the effects of the present invention.

The compounding ratio of the chromene compound in the hair-restoring preparation for external use of the present invention is generally about 0.0001 to 10 %, and preferably about 0.001 to 5 % of the total weight of the hair-restoring preparation for external use.

### Hair-restoring preparation for oral use

The hair-restoring preparation for oral use of the present invention is an oral preparation containing the hair growth stimulant of the present invention. The hair-restoring preparation for oral use of the present invention can be obtained by forming the hair growth stimulant of the present invention by itself, or by suitably mixing it with known ingredients generally used for oral drugs, into preparations.

There are no limitations on the form of the hair-restoring preparation for oral use of the present invention, and it may be formed into tablets, pulvis, granules, capsules, liquids, etc.

In addition to the hair growth stimulant of the present invention, the hair-restoring preparation for oral use of the present invention may contain other ingredients that enhance hair regrowth and/or hair-nourishment effects. Examples of ingredients that enhance hair regrowth and/or hair-nourishment effects include finasteride, cepharanthine, etc.

The hair-restoring preparation for oral use of the present invention may further comprise one or more ingredients generally used for oral preparations selected from the group consisting of nutrients, excipients, preservatives, emulsifiers, solubilizing agents, in such an amount that they do not adversely affect the effects of the present invention.

The compounding ratio of the chromene compound in the hair-restoring preparation for oral use of the present invention is generally about 0.001 to 50 %, and preferably about 0.005 to 20 % of the total weight of the hair-restoring preparation for oral use.

The intake of the hair-restoring preparation for oral use of the present invention calculated as chromene compound weight is generally about 0.0001 to 5 g, and preferably about 0.001 to 1 g per adult per day.

### Hair-restoring food

The hair-restoring food of the present invention is a food that contains the hair growth stimulant of the present invention. The hair-restoring food of the present invention can be obtained by processing the hair growth stimulant of the present invention by itself into a food, or by suitably mixing it with known foods or food materials.

There are no limitations on the form of the food of the present invention. The food of the present invention can be prepared in a routine manner. The hair growth stimulant of the present invention may be mixed with suitable food materials or foods and processed into various forms such as powders, blocks, liquids, syrups, jellies, etc. The hair growth stimulant of the present invention may be added to foods of various forms such as powders, blocks, liquids, syrups and jellies.

Examples of foods of the present invention include soft drinks, juices, various types of tea and other beverages (energy drinks); powdered juices, powdered soups and like powdered beverages; cookies, biscuits, cereals, chewing gums, candies, gummy candies, tablets, wafers, senbei (rice cracker) and like snacks.

The food of the present invention may comprise other ingredients typically added to foods in such an amount that they do not adversely affect the effects of the present invention. Examples of such other ingredients include various nutrients, animal and plant derived components, excipients, bulking agents, sweeteners, flavoring agents, coloring agents, preservatives, emulsifiers, solubilizing agents, polyhydric alcohols and ester derivatives thereof; organic acids, inorganic acids, and salts thereof; water-soluble polymers.

The compounding ratio of the chromene compound in the hair-restoring food of the present invention is about 0.0001 to 5 %, and preferably about 0.001 to 2 % of the total weight of the hair-restoring food.

The intake of the hair-restoring food of the present invention calculated as chromene compound weight is generally about 0.00001 to 0.5 g, and preferably about 0.0001 to 0.1 g per adult per day.

The hair growth stimulant of the present invention contains at least one type of a chromene compound represented by Structural Formula (1) as an active ingredient. The chromene compound achieves excellent hair restoration effects such as prevention of hair loss and promotion of hair regrowth. The chromene compound is a very safe material. Therefore, the hair growth stimulant of the present invention is suitable for use as a hair growth stimulant with a high level of safety that achieves excellent hair restoration effects.

The hair-restoring preparation for external use, hair-restoring preparation for oral use and hair-restoring food containing the hair growth stimulant of the present invention are also very safe and are suitable for use as a preparation for external use, oral preparation and food achieving excellent hair restoration effects, such as prevention of hair loss and promotion of hair regrowth.

### BEST MODE FOR CARRYING OUT THE INVENTION

The present invention is explained in detail below with reference to Examples. However, the present invention is not limited to these Examples.

### Experimental Example 1: Obtaining chromene compounds

Chromene compounds were obtained and identified by the method disclosed in a document prepared by Shibuya, et al. (Chem. Pharm. Bull., 47(5), 695-698, 1999). The method is described below.

Dried leaves of kumis kuching (800 g), which is a plant of the genus *Orthosiphon* in the family Lamiaceae, were subjected to extraction using boiling water, obtaining an extract. The thus-obtained extract was subjected to solvent fractionation using chloroform and water as solvents, obtaining a chloroform-soluble portion and a water-soluble portion. A chloroform-soluble portion and water-soluble portion were obtained in proportions of 2.3% and 25% respectively relative to the total weight of kumis kuching dried leaves. The chloroform-soluble portion was isolated and purified using silica gel oven column chromatography (column (SiO₂), solvent (chloroform : methanol = 100 : 1 → methanol)), and then, HPLC (column (YMC-Pack SIL, (manufactured by YMC Co., Ltd.), solvent (n-hexane : ethyl acetate = 2 : 1)), obtaining three types of chromene compounds, i.e., methylripariochromene A, orthochromene A, and acetovanillochromene.

Identification of each compound was conducted by measuring ¹H-NMR spectra, ¹³C-NMR spectra, IR and UV spectra, and specific rotations.

The isolated yields were 0.63% (methylripariochromene A), 0.02% (orthochromene A), and 0.03% (acetovanillochromene), relative to the total weight of dried kumis kuching leaves.

### Test Example 1: Experiment for evaluating proliferation effect of cultured hair-matrix cells

Mouse hair tissues were collected and hair-matrix cells were cultured by the method disclosed by Tanigaki, et al., using serum free media (Tanigaki, et al., (1990), Arch. Dermatol. Res. 282: 402-407). Each chromene compound obtained in Experimental Example 1 was dissolved in a culture medium (Keratinocyte SFM (manufactured by GIBCO)) in such a manner that the resulting solution had a concentration of 1 or 10 µg/ml.

On the first day after the start of culturing hair-matrix cells, the culture media were changed to those containing the above-mentioned chromene compounds.

After culturing the hair-matrix cells for 6 days, they were separated from the culture dishes and the number of cells was counted. The effect of each chromene compound is shown by a relative ratio with the number of cells in the control group (the group cultured in a serum-free medium) being defined as 100%. Table 1 shows the results.

**Table 1**

| Sample | Concentration of the sample placed in culture medium (µg/ml) | Relative value of the number of cells (%control) | Proliferation rate (%control) |
|---|---|---|---|
| Culture medium only (control group) | | 100 | |
| Methylripariochromene A | 1 | 112 | 12 |
| Methylripariochromene A | 10 | 153 | 53 |
| Orthochromene A | 1 | 118 | 18 |
| Orthochromene A | 10 | 144 | 44 |
| Acetovanillochromene | 1 | 133 | 33 |
| Acetovanillochromene | 10 | 135 | 35 |

As shown in Table 1, it was confirmed that the chromene compound has excellent effects in promoting the proliferation of hair-matrix cells.

### Example 1: Formulation of preparation for external use

Ingredients were mixed in the proportions shown below and made into a hair tonic in a conventional manner.

| Ingredients | Compounding ratio (%) |
|---|---|
| Methylripariochromene A | 0.1 |
| 95% Ethanol | 70 |
| Solubilizing agent (polyoxyethylene hydrogenated castor oil [60EO]) | 1 |
| Propylene glycol | 5 |
| Fragrance | Trace |
| Water | Balance |

### Example 2: Formulation of preparation for external use

Ingredients were mixed in the proportions shown below and made into a hair lotion in a conventional manner.

| Ingredients | Compounding ratio(%) |
|---|---|
| Hydroxyethyl cellulose | 0.4 |
| Ethanol | 5 |
| Butan-1,3-diol | 5 |
| Paraoxybenzoate | 0.2 |
| Orthochromene A | 0.1 |
| Fragrance | Trace |
| Water | Balance |

### Example 3: Formulation of preparation for external use

Ingredients were mixed in the proportions shown below and made into a hair cream in a conventional manner.

| Ingredients | Compounding ratio (%) |
|---|---|
| Beeswax | 5 |
| Lanolin | 4 |
| Vaseline | 5 |
| Liquid paraffin | 33 |
| Emulsifier (Polyoxyethylene sorbitan monostearate (20EO)) | 4 |
| Paraoxybenzoate | 0.2 |
| Fragrance | Trace |
| Methylripariochromene A | 0.01 |
| Borax | 1 |
| Water | Balance |

### Example 4: Formulation of preparation for oral use

Ingredients were mixed in the proportions shown below and made into sugar-coated tablets in a conventional manner.

| Ingredients | Compounding ratio (%) |
|---|---|
| Dolomite (Calcium 20%, Magnesium 10%) | Balance |
| Powdered reduced malt sugar syrup | 20 |
| Lactose | 17 |
| Sucrose fatty acid ester | 3 |
| Methylripariochromene A | 0.005 |

### Example 5: Preparation of food

Ingredients were mixed in the proportions shown below and made into tablets in a conventional manner.

| Ingredients | Compounding ratio(%) |
|---|---|
| Dextrin | Balance |
| Powdered reduced malt sugar syrup | 20 |
| Lactose | 20 |
| Trehalose | 10 |
| Orthochromene A | 0.005 |
| Aspartame | Trace |
| Flavor | Trace |

### Example 6: Preparation of food

Ingredients were mixed in the proportions shown below and made into a beverage in a conventional manner.

| Ingredients | Compounding ratio(%) |
|---|---|
| Orthochromene A | 0.001 |
| Sucrose | 2 |
| Ascorbic acid | 1.5 |
| Preservatives | Trace |
| Flavor | Trace |
| Purified water | Balance |

### INDUSTRIAL APPLICABILITY

The hair growth stimulant of the present invention can be suitably used for preventing hair loss, promoting hair regrowth and/or hair-nourishment, etc.

The hair-restoring preparation for external use, hair-restoring preparation for oral use, and hair-restoring food containing the hair growth stimulant of the present invention can also be suitably used for preventing hair loss, promoting hair regrowth and/or hair-nourishment, etc.

## Claims

1. A hair growth stimulant comprising at least one chromene compound represented by Structural Formula (1) as an active ingredient: 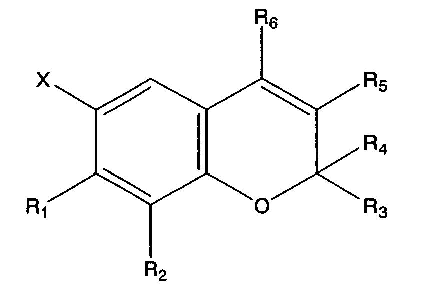 wherein R₁ and R₂ are each independently -H, -OH, or -OR wherein R is a C₁₋₄ alkyl group; R₃ and R₄ are each independently -H, or a C₁₋₄ alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group.

2. The hair growth stimulant according to Claim 1,
wherein the chromene compound represented by Structural Formula (1) is at least one member selected from the group consisting of methylripariochromene A (6-acetyl-7,8-dimethoxy-2,2-dimethylchromene), acetovanillochromene (6-acetyl-8-methoxy-2,2-dimethylchromene), and orthochromene A (6-(1-hydroxyethyl)-7,8-dimethoxy-2,2-dimethylchromene).

3. The hair-restoring preparation for external use comprising the hair growth stimulant of Claim 1 or 2.

4. The hair-restoring preparation for oral use comprising the hair growth stimulant of Claim 1 or 2.

5. The hair-restoring food comprising the hair growth stimulant of Claim 1 or 2.

6. A method for preparing a hair growth stimulant containing at least one chromene compound represented by Structural Formula (1) as an active ingredient, 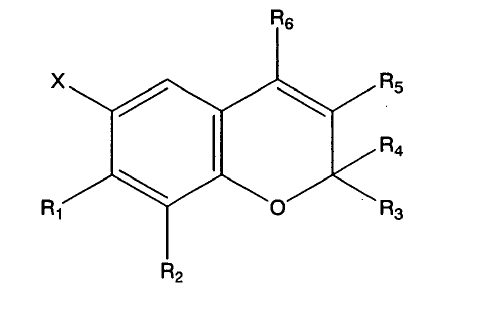 wherein R₁ and R₂ are each independently -H, -OH, or -OR wherein R is a C₁₋₄ alkyl group; R₃ and R₄ are each independently -H, or a C₁₋₄ alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group; the method comprising steps (i) and (ii):
(i) obtaining a chromene compound-containing extract by contacting a solvent with at least one plant selected from the group consisting of plants of the genus *Orthosiphon* in the family Lamiaceae, plants of the genus Eupatorium in the family Asteraceae, and plants of the genus Stevia in the family Asteraceae; and
(ii) isolating a chromene compound represented by Structural Formula (1) from the extract obtained in (i).

7. The method for preparing a hair growth stimulant according to Claim 6, wherein the chromene compound represented by Structural Formula (1) is at least one member selected from the group consisting of methylripariochromene A (6-acetyl-7,8-dimethoxy-2,2-dimethylchromene), acetovanillochromene (6-acetyl-8-methoxy-2,2-dimethylchromene), and orthochromene A (6-(1-hydroxyethyl)-7,8-dimethoxy-2,2-dimethylchromene).

8. A hair-restoring method comprising the step of contacting an effective amount of a hair growth stimulant containing at least one chromene compound represented by Structural Formula (1) as an active ingredient with hair tissue: 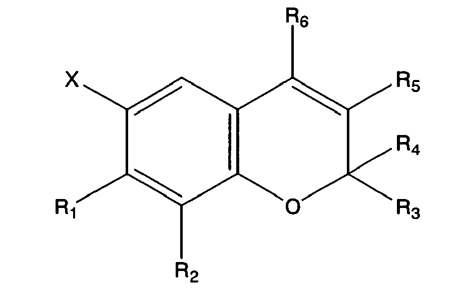 wherein R₁ and R₂ are each independently -H, -OH, or -OR wherein R is a C₁₋₄ alkyl group; R₃ and R₄ are each independently -H, or a C₁₋₄ alkyl group; R₅ and R₆ are each independently -H, a C₁₋₄ alkyl group, or an optionally substituted phenyl group; and X is - CH(OH)R₇, or -C(=O)R₇ wherein R₇ is a C₁₋₄ alkyl group or an optionally substituted phenyl group.
